# EUROPÄISCHE PATENTSCHRIFT

(11) **EP 2 490 753 B1**
(45) Veröffentlichungstag und Bekanntmachung des Hinweises auf die Patenterteilung: **11.10.2017**
(21) Anmeldenummer: 10779259.0
(22) Anmeldetag: 22.10.2010
(51) Int. Cl.: A61M 39/16, A61M 39/22, F16K 11/085, F16K 27/06

(54) **MEHRWEGEHAHN MIT ANTISEPTISCH BZW. ANTIMIKROBIELL WIRKENDEN OBERFLÄCHEN**
SELECTOR VALVE HAVING ANTISEPTICALLY AND/OR ANTIMICROBIALLY ACTING SURFACES
ROBINET À PLUSIEURS VOIES PRÉSENTANT DES SURFACES À ACTION ANTISEPTIQUE OU ANTIMICROBIENNE

(30) Priorität: 23.10.2009 DE 202009013408 U
(43) Veröffentlichungstag der Anmeldung: 29.08.2012
(73) Patentinhaber: Hopf, Hans-Jürgen, 90513 Zirndorf (DE)
(72) Erfinder: HOPF, Hans-Jürgen, 90513 Zirndorf (DE); HOPF, Michael, 90513 Zirndorf (DE); HOPF, Alexander, 90491 Nürnberg (DE)
(74) Vertreter: Wittmann, Ernst-Ulrich
(86) Internationale Anmeldenummer: PCT/EP2010/065950
(87) Internationale Veröffentlichungsnummer: WO 2011/048204

(56) Entgegenhaltungen:
- WO-A1-94/22522
- DE-U1- 20 005 691
- GB-A- 1 344 166

## Beschreibung

Die vorliegende Erfindung betrifft einen Mehrwegehahn, wie er insbesondere in der Medizin eingesetzt wird.

Zum Beispiel sind im Stand der Technik Dreiwegehähne bekannt und werden insbesondere in der Medizin und Medizintechnik eingesetzt. Hierbei werden sie insbesondere im infusionsbereich, dem Bereich der künstlichen Ernährung, bei der Transfusion und insbesondere für die Zu- bzw. Überleitung von verschiedenen Durchflussmedien und als sogenanntes "Injektions-Equipment" für die medizinische und pharmazeutische Ausrüstung verwendet. Die Dreiwegehähne werden ferner auch in medizinischen Systemen verwendet, welche unter anderem aus mehreren Komponenten bestehen. Solche Systeme umfassen unter anderem Schwerkraftinfusionen, Pumpen oder Pumpüberleitungssysteme, Sondennahrungssysteme, Injektionen, Kombinationen hiervon und dergleichen.

GB 1 344 166 A offenbart einen Mehrwegehahn zur Verwendung an Infusionssets, bei dem das Stellglied in einer am Gehäuseboden angeordneten Rille geführt wird, welche durch eine zentrisch angeordnete Erhebung und die Gehäusewand gebildet wird, und so auch bei höheren auf das Stellglied wirkendem Druck in Position gehalten wird.

DE 200 05 691 U1 offenbart einen Mehrweghahn mit am Gehäuseboden angeordneten, in radialer Richtung zwischen Gehäuse und Küken wirkenden Rastelementen, mittels derer die Betriebsstellung des Kükens bei der Bedienung deutlich spürbar vorgegeben und auch fixiert wird.

Ein Dreiwegehahn kann ferner durch die Kombination mit mehreren Dreiwegehähnen zu einer sog. Mehrwegehahnbank oder "Manifold" (Mehrfachverteiler) montiert werden.

Nachteilig ist es jedoch, dass die im Stand der Technik bekannten Mehrweghähne im Gebrauch mehr oder weniger offen platziert werden, so dass diese insbesondere wenn keine Flüssigkeit dem Patienten appliziert wird, mit der Luft im Behandlungsraum bzw. sogar in Kontakt mit anderen Gegenständen und Personen gelangen und dadurch zum Beispiel mit Keimen verunreinigt werden. Diese Kontamination kann unter Umständen zu schwerwiegenden Infektionen bei den zu behandelnden Personen führen.

Aufgabe der vorliegenden Erfindung ist es, die im Stand der Technik bekannten Nachteile wenigstens teilweise zu überwinden.

Die vorstehende Aufgabe wird durch einen erfindungsgemäßen Mehrwegehahn gemäß Anspruch 1 gelöst. Bevorzugte Ausgestaltungsformen des Mehrwegehahns sind Gegenstand der Unteransprüche.

Der erfindungsgemäße Mehrwegehahn zum Einsatz in der Medizin und/oder Medizintechnik weist ein wenigstens abschnittsweise von einem Medium durchströmbares Grundgehäuse und ein in dem Grundgehäuse um eine zentrale Achse drehbeweglich aufgenommenes Stellglied auf. Der erfindungsgemäße Mehrwegehahn ist dadurch gekennzeichnet, dass er Anschlussstellen für den Zulauf und den Ablauf des Mediums aufweist.

Des Weiteren bildet das Grundgehäuse des erfindungsgemäßen Mehrwegehahns eine Stellgliedaufnahme, welche dadurch gekennzeichnet ist, dass sie einen konzentrisch zur zentralen Achse des Grundgehäuses angeordneten Zapfen, sowie eine an der Innenseite des Grundgehäuses radial umlaufende Aussparung ausweist. Das Stellglied des erfindungsgemäßen Mehrwegehahns weist ein Bedienelement, sowie einen im Wesentlichen hohlzylindrischen Abschnitt auf, der den Zapfen der Stellgliedaufnahme des Grundgehäuses wenigstens abschnittsweise aufnimmt, wenigstens eine Durchflussöffnung zur Fluidverbindung wenigstens zweier Anschlussstellen und an der Außenseite des hohlzylindrischen Abschnitts wenigstens eine radial umlaufende Ausformung aufweist.

Der Zapfen des Grundgehäuses des erfindungsgemäßen Mehrwegehahns ist so ausgebildet, dass er wenigstens im Bereich einer Anschlussstelle des Grundgehäuses ein Gegenlager für das Stellglied aufweist beziehungsweise bereitstellt, welches zusammen mit dem Stellglied eine Dichtfläche im Bereich wenigstens einer Anschlussstelle bildet.

Der erfindungsgemäße Mehrwegehahn ferner dadurch gekennzeichnet, dass wenigstens die mit der Flüssigkeit in Kontakt kommenden Oberflächen des Mehrwegehahns, insbesondere die innen liegenden Oberflächen des Mehrwegehahns wenigstens abschnittsweise aus einem Material hergestellt sind, welches antiseptische und/oder antibakteriell und/oder antimikrobielle Eigenschaften aufweist.

Solche antimikrobielle oder antiseptsche Materialen sind beispielsweise Materialien wie hochporöses Silber, ionenfrei hergestelltes Silber, Silberverbindungen, Kupfer, ionenfrei hergestelltes Kupfer, Kupferverbindungen und insbesondere MicroSilber, MicroKupfer, Metallionen freisetzende Verbindungen und ferner Compounds, in welchen unter anderem Silber-, Kupfer, und/oder Metallionen freisetzende Zusatzstoffe, insbesondere antiseptische, antibakterielle und/oder antimikrobielle Zusatzstoffe beigefügt sind, Kombinationen hiervon und dergleichen.

Als Compounds werden insbesondere Gemische aus im wesentlichen reinen Grundstoffen bezeichnet, welchen beispielsweise Füllstoffe, Verstärkungsstoffe und weitere Additive beigemischt werden und durch welche wenigstens zwei Stoffe miteinander zu einer im wesentlichen homogenen Mischung verbunden werden.

Vorzugsweise werden diese im Bereich der entsprechenden Oberflächen angeordnet, wobei gemäß einer weiteren besonders bevorzugten Ausführungsform die entsprechenden Materialien auch in dem Kunststoff, aus welchem der Mehrwegehahn bzw. dessen Bestandteile hergestellt sind eingebunden sind. So kann insbesondere ein hochporöses Silber, welches vorzugsweise auch im wesentlichen ionenfrei hergestellt ist, in den Kunststoff eingemischt werden, wobei hieraus dann der Grundkörper und/oder das Stellglied wenigstens abschnittsweise hergestellt wird. Alternativ liegt es auch im Sinn der vorliegenden Erfindung die Oberflächen mit einem entsprechenden Material bzw. Materialkombination zu beschichten.

Gemäß einer weiteren besonders bevorzugten Ausführungsform ist der erfindungsgemäße Mehrwegehahns dadurch gekennzeichnet, dass der Zapfen des Grundgehäuses wenigstens eine Abflachung am Umfang zur zentralen Achse aufweist und die Ausformung des Stellglieds in die Aussparung der Stellgliedaufnahme des Grundgehäuses zur formschlüssigen und vorzugsweise fluiddichten Fixierung des Stellglieds in der Stellgliedaufnahme eingreift.

Unter Medium im Sinne der vorliegenden Erfindung werden alle Arten von Fluiden, insbesondere Flüssigkeiten, wie beispielsweise wässrige Flüssigkeiten, Blut, Ernährungsbrei, etc. und auch Gase verstanden.

Unter dem Grundgehäuse im Sinne der vorliegenden Erfindung wird ein Bauteil des Mehrwegehahns, welcher das Gehäuse mit den Anschlussstellen und der Stellgliedaufnahme aufweist verstanden, wobei unter letzterer der Bereich des Grundgehäuses verstanden wird, welcher zur Aufnahme des Stellglieds dient.

Unter Stellglied im Sinne der vorliegenden Erfindung wird ein, wenigstens eine Strömungsöffnung abschließendes sogenanntes "Kücken" verstanden. Das Bedienelement im Sinne der vorliegenden Erfindung ist ein mit dem Stellglied fest verbundenes Element, wie zum Beispiel ein Hahn, mit dem sich das Stellglied um die zentrale Achse drehen lässt. Dieser kann einen oder mehrere, üblicherweise zwei, drei oder vier Stellhebel aufweisen.

Unter einer Abflachung am Umfang des Zapfens wird eine Veränderung des kreisrunden Umfangs des Zapfens in Form einer Kreissehne in der Draufsicht verstanden, wobei die Abflachung eine entsprechende Fläche bildet.

In einer weiteren Ausführungsform des erfindungsgemäßen Mehrwegehahns, weist das Grundgehäuse zwei, drei oder vier Anschlussstellen auf.

Gemäß einer weiteren, besonders bevorzugten Ausführungsform ist das den Mehrwegehahn durchströmende Medium Flüssigkeiten, insbesondere ausgewählt aus einer Gruppe, welche Injektionslösungen, Infusionslösungen, Nährlösungen, Blut, Plasma sowie Kombinationen hieraus und dergleichen aufweist.

Gemäß einer weiteren, besonders bevorzugten Ausführungsform weist die den Mehrwegehahn durchströmende Flüssigkeit eine Viskosität auf, welche 0,7 mPa s⁻¹ und 10⁶ mPa s⁻¹, bevorzugt zwischen 1 mPa s⁻¹ und 10⁵ mPa s⁻¹ und besonders bevorzugt bei etwa 10² mPa s⁻¹ liegt.

Gemäß einer weiteren Ausführungsform des erfindungsgemäßen Mehrwegehahns, ist der Zapfen des Grundgehäuses wenigstens abschnittsweise konisch ausgeführt. In einer weiteren, besonders bevorzugten Ausführungsform sind konisch und nicht konische und/oder konische Abschnitte mit verschiedenen Konuswinkel in stufiger Abfolge ausgeführt. Der Konuswinkel gemäß der vorliegenden Situation wird als Abweichung von der zentralen Achse verstanden; insbesondere werden positive und negative Winkel unter Konuswinkel verstanden. In einer weiteren, besonders bevorzugten Ausführungsform weist der Zapfen eine alternierende Folge positiver und negativer Konuswinkel auf. In einer weiteren bevorzugten Ausführungsform weist der Zapfen relativ zur zentralen Achse nach innen oder außen versetzte, insbesondere konische Abschnitte auf.

Gemäß einer weiteren bevorzugten Ausführungsform ist die Innenseite des hohlzylindrischen Abschnitts wenigstens abschnittsweise konisch ausgeführt und ist insbesondere an den konischen Verlauf des Zapfens im Wesentlichen angepasst.

Gemäß der vorliegenden Erfindung sind die Durchflussöffnungen des Stellglieds torförmig ausgeführt, insbesondere derartig ausgeführt, dass die Durchflussöffnung zum Ende des hohlzylindrischen Abschnitts des Stellglieds offen ist. Ferner wird insbesondere die Durchflussöffnung vorzugsweise in Abhängigkeit des zu verwendenden Fluids in ihrer Größe angepasst, wobei ferner auch eine Kombination der Form des Gegenlagers wie beispielsweise konisch und rechteckig mit berücksichtigt wird.

Gemäß einer weiteren, besonders bevorzugten Ausführungsform des erfindungsgemäßen Mehrwegehahns entspricht die Zahl der Durchflussöffnungen des Stellglieds der Zahl der Anschlussstellen am Grundgehäuse. So weist zum Beispiel eine Ausführungsform mit zwei Anschlussstellen zwei Durchflussöffnungen auf. Gemäß einer weiteren bevorzugten Ausführungsform weist das Stellglied weniger Durchlassöffnungen auf, als Anschlussstellen am Grundgehäuse vorhanden sind.

Gemäß einer weiteren, besonders bevorzugten Ausführungsform des erfindungsgemäßen Mehrwegehahns weist der hohlzylindrische Abschnitt des Stellglieds zwei, drei oder vier radial zur zentralen Achse umlaufende Ausformungen auf und die Innensite der Stellgliedaufnahme weist eine entsprechende Zahl an Aussparungen auf.

Gemäß einer weiteren, besonders bevorzugten Ausführungsform des Mehrwegehahns gemäß der vorliegenden Erfindung ist die Mitte der Abflachung des Zapfens in der Stellgliedaufnahme des Grundgehäuses um 30° bis 60°, insbesondere um 45° versetzt zur Anschlussstelle des Grundgehäuses angeordnet, wobei die seitlichen Ränder der Abflachung ± 20° ± 1°, vorzugsweise ± 15 ± 2° und über etwa ± 10° von der Mitte der Abflachung versetzt angeordnet sind.

Gemäß einer weiteren, besonders bevorzugten Ausführungsform des erfindungsgemäßen Mehrwegehahns wird das Gegenlager für das Stellglied durch einen nicht abgeflachten Bereich des Zapfens gebildet. Die Modifikation der Gegenlageraufnahme durch die erfindungsgemäße Abflachung oder Abflachungen am Zapfen führt zu einer Reduktion der Reibungsfläche zwischen Zapfen und Stellglied. Dadurch wird eine Leichtgängigkeit (Bedienerfreundlichkeit) des Mehrwegehahns gewährleistet und gleichzeitig sichergestellt, dass der Hahn nicht durch das eingesetzte, insbesondere viskose Medium, aus seiner definierten, eingestellten Position verschoben werden kann.

Unter Gegenlager im Sinne der vorliegenden Erfindung wird ein Bereich der Stellgliedaufnahme verstanden, der den Druck auf das Stellglied, insbesondere in geschlossenem Zustand, aufnimmt, bzw. einer Verformung des Stellglieds unter dem Druck des Mediums entgegen wirkt.

Gemäß einer weiteren, besonders bevorzugten Ausführungsform des Mehrwegehahns gemäß der vorliegenden Erfindung, bilden die radial umlaufende Ausformung am hohlzylindrischen Abschnitt des Stellglieds und die Aussparung an der Innenseite der Stellgliedaufnahme eine Fügeverbindung, wobei die Fügeverbindung insbesondere einen Fügering am Gehäuse aufweist. Der Fügering weist gemäß einer weiteren bevorzugten Ausführungsform einen radial umlaufenden Dichtabschnitt und/oder einen Positionierabschnitt auf. Insbesondere ist die Fügeverbindung zwischen dem Stellglied und dem Grundgehäuse so gestaltet, dass durch die Verbindung der beiden Teile eine Dichtheit von größer als 4 bar erreicht wird.

Gemäß einer weiteren bevorzugten Ausführungsform des erfindungsgemäßen Mehrwegehahns wird wenigstens das Gehäuse und/oder das Stellglied wenigstens teilweise aus einem Material hergestellt, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethylmetaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Bisphenol-A-freie Materialien wie zum Beispiel Tritan, Kombinationen hiervon und dergleichen umfasst.

Die Aufgabe der vorliegenden Erfindung wird ferner auch durch ein Verfahren zur Herstellung eines Mehrwegehahns gelöst, welches wenigstens die folgenden Schritte aufweist. In einem vorzugsweise ersten Schritt des erfindungsgemäßen Verfahrens wird das Grundgehäuse sowie das Stellglied hergestellt, wobei die Herstellung vorzugsweise im Spritzgussverfahren erfolgt. Nach der Aufnahme des Grundgehäuses in eine Montagevorrichtung gemäß des erfindungsgemäßen Verfahrens, wird der hohlzylindrische Abschnitt des Stellglieds in die Stellgliedaufnahme des Grundzylinders eingeführt und eingepresst. Der Einpressvorgang wird gemäß des erfindungsgemäßen Verfahrens mit Erreichen einer vorbestimmten Einpresstiefe beendet. Die Einpresstiefe wird insbesondere durch einen definierten Anschlag bestimmt. Die Dichtheit des erfindungsgemäßen Hahns bestimmt sich insbesondere durch die definierte Einpresstiefe. So wird die Dichtheit im oberen Bereich durch die definierte Einpresstiefe in den Fügerand erreicht. Im Inneren des Gehäuses wird die Dichtheit zu den entsprechenden Durchflussöffnungen über die Einpresstiefe und damit über das konische Gegenlager sichergestellt.

Gemäß einer weiteren, besonders bevorzugten Ausführungsform ist der die Einpresstiefe bestimmende Anschlag Bestandteil der umlaufenden Ausformung des hohlzylindrischen Abschnitts des Stellglieds und/oder Bestandteil der Stellgliedaufnahme des Grundgehäuses; insbesondere ist der Anschlag Bestandteil der Fügeverbindung.

Gemäß einer weiteren besonders bevorzugten Ausführungsform ist der das Gegenlager bildende Zapfen wenigstens abschnittsweise hohl ausgeführt oder ist gemäß einer weiteren besonders bevorzugten Ausführungsform aus Vollmaterial hergestellt.

Gemäß einer weiteren, besonders bevorzugten Ausführungsform handelt es sich bei den beim Fügen der Bauteile gemäß des erfindungsgemäßen Verfahrens auftretenden Verformungen im Wesentlichen um elastische Verformungen.

Die vorliegende Erfindung umfasst ferner auch die Verwendung des erfindungsgemäßen Mehrwegehahns in der Medizin und Medizintechnik, insbesondere für die Zu- und Überleitung von verschiedenen Durchflussmedien, insbesondere für die Schwerkraftinfusion, für Pumpüberleitungssysteme, Sondennahrungssysteme, Injektionen sowie Kombinationen hiervon und dergleichen.

Die Erfindung wird nachfolgend anhand eines bevorzugten Ausführungsbeispiels erläutert, wobei darauf hingewiesen wird, dass durch dieses Beispiel Abwandlungen beziehungsweise Ergänzungen wie sie sich für den Fachmann unmittelbar ergeben mit umfasst sind. Darüber hinaus stellt dieses bevorzugte Ausführungsbeispiel keine Beschränkung der Erfindung in der Art dar, dass Abwandlungen und Ergänzungen im Umfang der vorliegenden Erfindung liegen.

Dabei zeigen:
Fig. 1 eine Aufsicht auf ein Grundgehäuse eines Dreiweghahns;
Fig. 2 einen Querschnitt durch das Grundgehäuse aus Fig. 1 in Richtung der B-B-Schnittlinie;
Fig. 3 einen Querschnitt durch das Grundgehäuse aus Fig. 1 in Richtung der A-A-Schnittlinie;
Fig. 4 eine Frontalansicht des Grundgehäuses aus Fig. 1;
Fig. 5 eine Detaildarstellung der radial umlaufenden Aussparung der Stellgliedaufnahme des Grundgehäuses;
Fig. 6 ein Stellglied eines erfindungsgemäße Mehrweghahns;
Fig. 7 einen Teilquerschnitt durch das Grundgehäuse aus Fig. 1 in Richtung der A-A- Schnittlinie mit eingepresstem Stellglied.

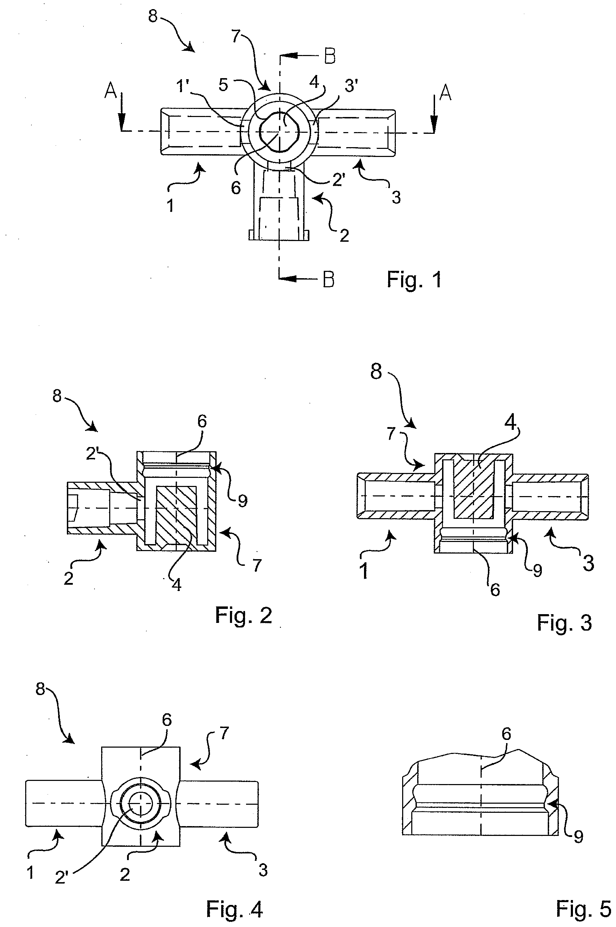

Das Ausführungsbeispiel gemäß Figur 1 zeigt das Grundgehäuse 8 mit drei Anschlussstellen 1, 2, 3 und damit korrespondierenden Durchlassöffnungen 1', 2', 3' in der Stellgliedaufnahme 7. In der Stellgliedaufnahme 7 ist um eine zentrale Achse 6 ein Zapfen 4 mit vier Abflachungen 5 angeordnet. Die unterbrochenen Linien markieren die Schnittlinien A-A und B-B für die Querschnitte in den Figuren 2 und 3. So zeigt Figur 2 einen Querschnitt des Grundgehäuses in Richtung der B-B-Schnittlinie. In der Stellgliedaufnahme 7 ist um die zentrale Achse 6 der Zapfen 4 angeordnet. Die Anschlussstelle 2 ist mit der Stellgliedaufnahme verbunden, wobei die Durchlassöffnung 2' gebildet wird. Die Innenseite der Stellgliedaufnahme 6 weist eine radial umlaufende Aussparung 9 auf, wobei die Aussparung 9 so gestaltet ist, dass sie unterschiedliche Tiefen aufweist.

Figur 3 zeigt einen Querschnitt des Grundgehäuses gemäß Figur 2 in Richtung der in Figur 1 markierten A-A- Schnittlinie. Die beiden Anschlussstellen 1 und 3 sind mit der Stellgliedaufnahme verbunden, wobei die Durchlassöffnungen 1' und 3' gebildet werden. Die Innenseite der Stellgliedaufnahme 7 weist eine radial umlaufende Aussparung 9 auf, wobei die Aussparung 9 so gestaltet ist, dass sie unterschiedliche Tiefen aufweist.

Figur 4 zeigt eine Seitenansicht von vorn des Grundgehäuses entsprechend der Figur 1. Die Anschlussstellen 1, 2, 3 sind mit der Stellgliedaufnahme 7 verbunden. In der Mitte dieser Verbindungen befinden sich die entsprechenden Durchlassöffnungen, wie die Durchlassöffnung 2' in der Anschlussstelle 2.

Figur 5 zeigt einen Teil der Stellgliedaufnahme 7 mit der umlaufenden Aussparung 9 an ihrer Innenseite. Die Aussparung weist verschiedene Tiefen auf, so dass eine Abfolge von Vertiefungen und Auswölbungen gebildet wird.

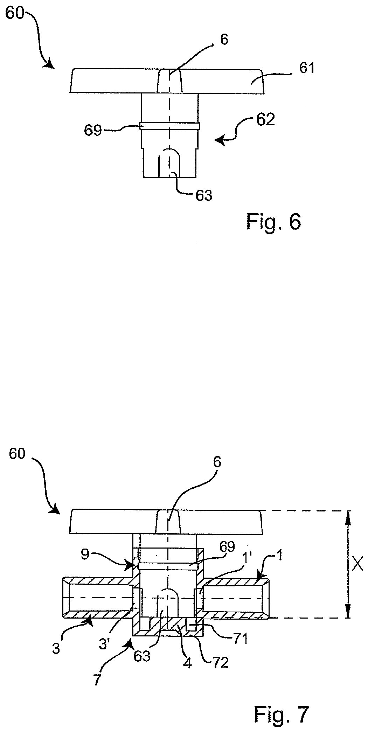

Figur 6 zeigt eine Ausführungsform eines Stellglied 60 für die Verwendung in Verbindung mit dem Grundgehäuse gemäß Figur 1. Das Stellglied weist ein Bedienelement 61 in Form eines Hahnes mit drei Stellhebeln auf, welches fest mit einem hohlzylindrischen Abschnitt 62 verbunden ist. Der hohlzylindrische Abschnitt ist konzentrisch um eine zentrale Achsen 6 angeordnet. An dem Bedienelement gegenüberliegenden Ende des hohlzylindrischen Abschnitts sind torförmige Durchlassöffnungen 63 ausgebildet. Die Zahl der Durchlassöffnungen 63 des Stellglieds entspricht der Zahl der Anschlussstellen des Grundgehäuses. Am hohlzylindrischen Abschnitt 62 des Stellglieds 60 ist eine radial umlaufende Ausformung 69 angeordnet.

Figur 7 zeigt die Verbindung des Grundgehäuses (schraffiert dargestellt) entsprechend Figur 1 mit dem Stellglied 60 entsprechend der Figur 6 als eine Ausführungsform des erfindungsgemäßen Mehrweghahns. Das Stellglied 60 ist bis zu einer vorbestimmten Einpresstiefe X in die Stellgliedaufnahme 7 des Grundgehäuses eingepresst. In dieser Position greift die Ausformung 69 des hohlyzylindrischen Abschnitts des Stellglieds 60 in die Aussparung 9 an der Innenseite der Stellgliedaufnahme 7 des Grundgehäuses. Der hohlzylindrische Abschnitt des Stellglieds 60 umfasst den um eine zentrale Achse 6 angeordnete Zapfen 4 der Stellgliedaufnahme 7 und bildet so ein Gegenlager für das Stellglied 60. Der Durchfluss des Mediums im geöffneten Zustand des Mehrweghahns erfolgt von einer der Anschlussstellen 1, 3 durch die zugehörige Durchlassöffnung des Grundgehäuses 1', 3' durch eine torförmige Durchlassöffnung des Stellglieds 63 über einen Freiraum 71 zwischen dem Stellglied 60 und dem Boden 72 der Stellgliedaufnahme 7 hin zu einer anderen Durchlassöffnung 63 des Stellglieds und durch die jeweils andere Durchlassöffnung 1', 3' des Grundgehäuses aus der jeweils anderes Anschlussstelle 1, 3 am Grundgehäuses hinaus. Die Dichtheit im oberen Bereich wird durch eine definierte Einpresstiefe des Stellglieds 60 in den Fügerand erreicht. Im Inneren des Grundgehäuses 8 wird die Dichtheit zu den entsprechenden Durchlassöffnungen 1, 2, 3 über das konische Gegenlager, gebildet durch den Zapfen 4, sichergestellt. Die Leichtgängigkeit des Hahns, also die leichte Beweglichkeit des Stellglieds 60 in der Stellgliedaufnahme 7 wird durch vier Abflachungen 5 am Zapfen 4 sichergestellt.

## Patentansprüche

1. Mehrwegehahn für den Einsatz in der Medizintechnik, insbesondere für den Einsatz bei der parenteralen oder enteralen Verabreichung von Flüssigkeiten, mit einem wenigstens abschnittsweise von der Flüssigkeit durchströmbaren Grundgehäuse (8) und einem hierin, um eine zentrale Achse (6) drehbeweglich aufgenommenen Stellglied (60), wobei das Grundgehäuse (8) Anschlussstellen (1, 2, 3) für den Zu- und Abfluss des Mediums aufweist und eine Stellgliedaufnahme (7) bildet, welche mit den Anschlussstellen (1,2,3) korrespondierende Durchlassöffnungen (1',2',3'), einen konzentrisch zur zentralen Achse (6) angeordneten Zapfen (4) und an der Innenseite des Grundgehäuses (8) eine radial umlaufende Aussparung (9) aufweist, und
das Stellglied (60) ein Bedienelement (61) und einen, im Wesentlichen hohlzylindrischen Abschnitt (62) aufweist, welcher den Zapfen (4) der Stellgliedaufnahme (7) wenigstens abschnittsweise aufnimmt, wenigstens eine Durchflussöffnung (63) zur Fluidverbindung wenigstens zweier Anschlussstellen (1,2,3) und eine, an der Außenseite des zylindrischen Abschnitts (62) wenigstens eine radial umlaufende Ausformung (69), aufweist und die Ausformung (69) des Stellglieds (60) in die Aussparung (9) der Stellgliedaufnahme (7) zur formschlüssigen und fluiddichten Fixierung des Stellglieds in der Stellgliedaufnahme eingreift, **dadurch gekennzeichnet, dass**
der Zapfen (4) sich vom Boden (72) der Stellgliedaufnahme über die Durchlassöffnungen (1',2', 3') des Grundgehäuses (8) erstreckt und ein Gegenlager für das Stellglied (60) zur Bildung einer Dichtfläche im Bereich wenigstens einer Anschlussstelle bildet und
die mit der Flüssigkeit in Kontakt kommende Oberfläche des Mehrwegehahns wenigstens abschnittsweise aus einem antiseptischen oder antimikrobiellen Material hergestellt werden.

2. Mehrwegehahn gemäß Anspruch 1, **dadurch gekennzeichnet, dass** der Zapfen (4) wenigstens eine Abflachung (5) am Umfang zur zentralen Achse (6) aufweist und die Ausformung (69) des Stellglieds in die Aussparung 9 der Stellgliedaufnahme (7) zur formschlüssigen und fluiddichten Fixierung des Stellglieds in der Stellgliedaufnahme (7) eingreift.

3. Mehrwegehahn gemäß Anspruch 1 oder 2, **dadurch gekennzeichnet, dass**
die Flüssigkeit aus einer Gruppe ausgewählt wird, welche Injektionslösungen, Infusionslösungen, Nährlösungen, BIut, Plasma, Gase, Luft Kombinationen hieraus und dergleichen aufweist.

4. Mehrwegehahn gemäß einem der vorstehenden Ansprüche, **dadurch gekennzeichnet, dass**
das antimikrobielle oder antiseptsche Material wenigstens ein keimtötendes Material aufweist, das aus einer Gruppe ausgewählt wird, welche hochporöses Silber, ionenfrei hergestelltes Silber, Silberverbindungen, Kupfer, ionenfrei hergestelltes Kupfer, Kupferverbindungen und insbesondere MicroSilber, MicroKupfer, Metallionen freisetzende Verbindungen und ferner Compounds, in welchen unter anderem Silber-, Kupfer, und/oder Metallionen freisetzende Zusatzstoffe, insbesondere antiseptische, antibakterielle und/oder antimikrobielle Zusatzstoffe beigefügt sind, Kombinationen hiervon und dergleichen umfasst.

5. Mehrwegehahn gemäß einem der vorstehenden Ansprüche, **dadurch gekennzeichnet, dass**
der Mehrwegehahn wenigstens teilweise aus einem Material hergestellt wird, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethylmetaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff- Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Bisphenol-A- freie Materialien wie zum Beispiel Tritan, Kombinationen hiervon und dergleichen umfasst.

## Claims

1. Selector valve for use in medical technology, in particular for use in parenteral or enteral administration of liquids, with a main housing (8) through which the liquid can flow at least in some sections, and with an actuator (60) accommodated in said main housing (8) and movable in rotation about a central axis (6), wherein the main housing (8) has attachment points (1, 2, 3) for the inflow and outflow of the medium and forms an actuator receptacle (7) which has through-openings (1', 2', 3') corresponding to the attachment points (1, 2, 3), a pin (4) arranged concentrically with respect to the central axis (6), and a radially circumferential recess (9) on the inside of the main housing (8), and the actuator (60) has an operating element (61) and a substantially hollowcylindrical portion (62) which accommodates the pin (4) of the actuator receptacle (7) at least in some sections, has at least one through-flow opening (63) for fluidically connecting at least two attachment points (1, 2, 3), and at least one radially circumferential protrusion on the outside of the cylindrical portion (62), and the protrusion (69) of the actuator (60) engages in the recess (9) of the actuator receptacle (7) in order to fix the actuator with form-fit engagement and in a leaktight manner in the actuator receptacle, **characterized in that** the pin (4) extends from the bottom (72) of the actuator receptacle across the through-openings (1', 2', 3') of the main housing (8) and forms a counterbearing for the actuator (60) in order to form a sealing surface in the area of at least one attachment point, and the surface of the selector valve coming into contact with the liquid is produced at least in some sections from an antiseptic or antimicrobial material.

2. Selector valve according to Claim 1, **characterized in that** the pin (4) has at least one flattening (5) at the circumference to the central axis (6), and the protrusion (69) of the actuator engages in the recess (9) of the actuator receptacle (7) in order to fix the actuator with form-fit engagement and in a leaktight manner in the actuator receptacle (7).

3. Selector valve according to Claim 1 or 2, **characterized in that** the liquid is chosen from a group comprising injection solutions, infusion solutions, nutrient solutions, blood, plasma, gases, air, combinations thereof and similar.

4. Selector valve according to one of the preceding claims, **characterized in that** the antimicrobial or antiseptic material has at least one germicidal material chosen from a group comprising highly porous silver, silver produced free of ions, silver compounds, copper, copper produced free of ions, copper compounds, and in particular micro silver, micro copper, compounds releasing metal ions, and moreover compounds to which are added *inter alia* additives that release silver, copper and/or metal ions, in particular antiseptic, antibacterial and/or antimicrobial additives, combinations thereof and similar.

5. Selector valve according to one of the preceding claims, **characterized in that** the selector valve is produced at least in some sections from a material chosen from a group comprising duroplastics and thermoplastics and in particular polyphenylene sulfide, polypropylene, poly-1-butene, polyvinyl chloride, polyvinylidene chloride, polymethyl methacrylate, polyacrylonitrile, polystyrene, polysulfone, polyacetal, polyvinyl alcohol, polyvinyl acetate, ionomers, fluoropolymer, polyethylene, polyamide, in particular a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenolic resin, urea resin, thiourea resin, melamine resin, alkyl resin, allyl resin, silicon, polyimide, polybenzimidazole, epoxy resin, casein plastic, crosslinked polyurethane, unsaturated polyester resin, materials free of bisphenol A, for example Tritan, combinations thereof and similar.

## Revendications

1. Robinet à plusieurs voies destiné à une utilisation dans la technologie médicale, notamment à une utilisation lors de l'administration parentérale ou entérale de liquides, comprenant un boîtier de base (8) qui peut au moins en partie être traversé par le liquide et un actionneur (60) logé dans celui-ci de manière mobile en rotation autour d'un axe central (6), le boîtier de base (8) comportant des points de raccordement (1, 2, 3) pour l'entrée et la sortie du milieu et formant un logement pour l'actionneur (7), qui comprend un tenon (4) agencé concentriquement à l'axe central (6) avec des ouvertures de passage (1', 2', 3') correspondant aux points de raccordement (1, 2 3) et un évidement (9) s'étendant radialement sur le pourtour de la face intérieure du boîtier de base (8), et
l'actionneur (60) comprenant un élément de commande (61) ainsi qu'une partie (62) ayant sensiblement la forme d'un cylindre creux qui reçoit au moins partiellement le tenon (4) du logement de l'actionneur (7), et qui comporte au moins un orifice d'écoulement (63), mettant en communication fluidique au moins deux points de raccordement (1, 2, 3), et au moins une protubérance (69) s'étendant radialement sur le pourtour de la face extérieure de la partie cylindrique (62), et
la protubérance (69) de l'actionneur (60) pénétrant dans l'évidement (9) du logement pour l'actionneur (7) pour la fixation par complémentarité de forme et étanche aux fluides de l'actionneur dans le logement pour l'actionneur,
**caractérisé en ce que**
le tenon (4) s'étend depuis le fond (72) du logement pour l'actionneur au-dessus des ouvertures de passage (1', 2', 3') du boîtier de base (8) et forme un palier de butée pour l'actionneur (60) permettant la formation d'une surface d'étanchéité dans la zone d'au moins un point de raccordement, et la surface du robinet à plusieurs voies qui entre en contact avec le liquide est réalisée au moins en partie dans un matériau antiseptique ou antimicrobien.

2. Robinet à plusieurs voies selon la revendication 1, **caractérisé en ce que** le tenon (4) comprend au moins un aplatissement (5) sur la périphérie de l'axe central (6) et la protubérance (69) de l'actionneur pénètre dans l'évidement du logement pour l'actionneur (7) pour la fixation par complémentarité de forme et étanche aux fluides de l'actionneur dans le logement pour l'actionneur (7).

3. Robinet à plusieurs voies selon la revendication 1 ou 2, **caractérisé en ce que** le liquide est choisi dans un groupe qui comprend les solutions pour injection, les solutions pour perfusion, les solutions nutritives, le sang, le plasma, les gaz, l'air, leurs combinaisons et analogues.

4. Robinet à plusieurs voies selon l'une quelconque des revendications précédentes, **caractérisé en ce que** le matériau antimicrobien ou antiseptique comprend au moins un matériau germicide, qui est choisi dans un groupe qui comprend l'argent hautement poreux, l'argent fabriqué sans ions, les composés d'argent, le cuivre, le cuivre fabriqué sans ions, les composés de cuivre et notamment le micro-argent, le micro-cuivre, les composés libérant des ions métalliques et également les composés dans lesquels des additifs libérant de l'argent, du cuivre et/ou des ions métalliques, entre autres, notamment des additifs antiseptiques, antibactériens et/ou antimicrobiens, sont ajoutés, leurs combinaisons et analogues.

5. Robinet à plusieurs voies selon l'une quelconque des revendications précédentes, **caractérisé en ce que** le robinet à plusieurs voies est réalisé au moins en partie dans un matériau qui est choisi dans le groupe qui comprend le polysulfure de phénylène, le polypropylène, le poly-1-butène, le polychlorure de vinyle, le polychlorure de vinylidène, le polyméthacrylate de méthyle, le polyacrylonitrile, le polystyrène, la polysulfone, le polyacétal, l'alcool polyvinylique, l'acétate de polyvinyle, les ionomères, les plastiques fluorés, le polyéthylène, le polyamide, notamment un polyamide partiellement aromatique, le polycarbonate, le polyester, l'oxyde de polyphénylène, la polysulfone, le polyvinylacétal, le polyuréthane et le polyéther chloré, le nitrate de cellulose, l'acétate de cellulose, l'éther de cellulose, la résine de phénol, la résine d'urée, la résine de thiourée, la résine de mélamine, la résine d'alkyle, la résine d'allyle, la silicone, le polyimide, le polybenzimidazole, la résine d'époxide, le plastique de caséine, le polyuréthane réticulé, la résine de polyester insaturée, les matériaux sans bisphénol A tels que par exemple le Tritan, leurs combinaisons et analogues.
